Europäisches Patentamt

European Patent Office

Office européen des brevets

(11) Publication number: **0 356 868**
**A2**

## (12) EUROPEAN PATENT APPLICATION

(21) Application number: 89115412.2

(22) Date of filing: 21.08.89

(51) Int. Cl.⁵: **A61K 6/02 , A61K 6/08**

(30) Priority: 01.09.88 US 239472'

(43) Date of publication of application:
**07.03.90 Bulletin 90/10**

(84) Designated Contracting States:
**AT BE CH DE ES FR GB GR IT LI LU NL SE**

(71) Applicant: DENTSPLY INTERNATIONAL, INC.
570 West College Avenue P.O. Box 872
York Pennsylvania 17405(US)

(72) Inventor: Chin-Teh, Huang
22 Crossley Drive
Dover, Delaware 19901(US)

(74) Representative: Wächtershäuser, Günter, Dr.
Tal 29
D-8000 München 2(DE)

(54) A method of treating a tooth with adhesive dental cavity basing composition.

(57) A method of treating a tooth comprising coating the tooth with a resin base composition which includes an adhesive is provided. The method provides a means by which a base material may be made resistant to displacement once it has been positioned. A base composition containing an adhesive, to be used in the method of the invention is also provided.

EP 0 356 868 A2

# A METHOD OF TREATING A TOOTH WITH ADHESIVE DENTAL CAVITY BASING COMPOSITION

## BACKGROUND

The invention relates to a method for treating a tooth comprising coating a tooth with resin compositions that include an adhesive promoting agent and optionally an elutable source of fluoride ions. Specific dental base compositions used in the method of the invention are also provided.

In the treatment of dental caries, it is often desirable, when the carious tissue has been removed, to coat the exposed dentin of the tooth with a liner or base composition prior to filling the tooth cavity with amalgam, composite or other filling material.

A base or liner composition is adapted to occlude dental tubulae, or at least minimize ingress of foreign material into dental tubulae, and to provide insulation or a barrier between the filling material and the tubulae. It has been found that such a barrier is desirable because, heat, cold, and other stimuli, when in contact with an uninsulated filling material, has an effect on the dental tubulae which may cause pain.

Because dental tubulae carry fluid through the dentin of the tooth, it is practically impossible to completely dry a prepared tooth cavity, because, as soon as the preparation is dried with a burst of air, for example, fluid from the dental tubulae, to a certain extent, replaces the fluid that has been removed. Because of this, it is difficult, if not impossible for prior art resin bases or liners to stick to the prepared cavity. The prior art bases and liners have a tendency to lift, and if the practitioner inadvertently displaces the base or liner before or while he places the filling material, he must again clean the cavity and again apply a fresh base or liner material.

The repetition of such procedures are inconvenient to the patient and have a detrimental affect on the efficiency of the practitioner's use of chair time.

It has been proposed, and is an object of this invention, that a liner or base composition that is adhesive may be adapted to bond to the dentin, and in turn may be desirably bonded to the filling material, and to occlude dental tubulae. Such a base composition may prevent some of the problems noted as being associated with prior art bases and dental liners.

It is the object of the present invention to provide base and liner compositions that do not lift off when inadvertently touched by the practitioner, and contain fluoride to help prevent the reoccurrence of dental caries.

## SUMMARY OF THE INVENTION

The present invention provides a method of treating a prepared tooth cavity comprising lining or basing the said tooth cavity with a dental resin composition which includes at least one adhesion promoting and optionally an elutable fluoride which may comprise sodium fluoride or strontium fluoride or other elutable ionic fluoride. Subsequently the cavity preparation and base liner are filled with an amalgam, composite, inlay or other filling material used to restore the anatomic form and function of the tooth.

Also provided are specific resin compositions adapted for use in the method of the invention.

## DETAILED DESCRIPTION OF THE INVENTION

The compositions of the invention, when used as a dental sealer or a liner base, comprise resin monomers, inorganic fillers, photoinitiator, reducing agent, an adhesion promoting composition and optionally, a source of elutable fluoride ions and a stabilizer.

In the method of the invention, the practitioner first removes carious tissue and prepares the cavity as is conventional in the art. When the cavity has been cleaned and dried, the practitioner then applies a small amount of the base or liner of the invention to the bottom of the cavity, and in a preferred embodiment cures the applied base or liner by the application of actinic light in the visible wavelength range using, for example, a PRISMA® LITE apparatus, a product of L. D. Caulk Company, a Division of Dentsply International Inc.

Those skilled in the art will recognize that an adhesive resinous base composition of the invention may be prepared, by means well known in the art, to be self-curing.

After curing the applied base or liner composition, the practitioner may inspect the cured base and determine, in the case of a deep cavity, whether additional base is needed. Since the base of the invention is a resin composition, additional base material may be added, and the base built-up, at the will of the practitioner.

After determining if the base is in the desired condition, the practitioner may then complete the treatment, for example, by filling the tooth cavity with filling material, as is conventional in the art.

The resin composition of the invention simplifies the method of applying the base and substantially eliminates the prior art problem of the lifting of the base. In a composition which comprises an elutable fluoride, wherein fluoride ions are extracted from the composition from the action of the fluids of the mouth (aqueous elutable) the fluoride acts to help prevent further carious activity in the tooth.

The source of elutable fluoride ions may be any suitable fluoride containing material. Particularly preferred is sodium fluoride. Strontium fluoride provides a suitable source of elutable fluoride ions and may be used when it is desirable to provide a radiopaque liner or base material as illustrated in U.S. patent applications Serial No. 034,880 and Serial No. 034,877 to Billington et al.

Exemplary of the resin monomers that can be used in the base/liner composition of the invention are low viscosity urethane methacrylate (U.M.), 2,2-bis-p-x-methacryloxy-B-hydroxypropoxyphenyl propane (Bis-GMA), triethylene glycol dimethacrylate (TEGDMA), and dipentaerythritol pentaacrylate phosphoric acid ester (PENTA - which also acts as an adhesive promoter).

PENTA is described in U.S. patent 4,514,342 to Billington et al, said patent being incorporated herein by reference. U.M. is generic to and embraces other urethane methacrylate deriviatives such as urethane dimethacrylate (U.D.M.A.). U.M. and U.D.M.A. (see Example 1) are described in U.S. patent application 120,269 filed November 13, 1987, said application being incorporated herein by reference.

PENTA has been found to provide a good adhesive bond to the inorganic structure of teeth and is believed to form a chemical bond with calcium ions in the tooth structure. In addition, the resin portion of the PENTA compound cross-links with the other resins in the composition on cure, to provide a base which bonds to a tooth and is structurally strong.

Those skilled in the art will recognize that adhesives which bond to the organic structure of the tooth, such as protein, may also be used in the base composition.

Also, diketo photoinitiators such as camphorquinone (CQ) are preferred, although those skilled in the art will recognize that titanate and phosphonate photoinitiators may also be used.

Preferred reducing agents are organic amines such as morpholinoethyl methacrylate (MEM), 4-ethyl diaminobenzoate (EDAB) and methyl diethanolamine (MDEA).

Preferred inorganic fillers are glasses known for use in dental compositions and other such fillers and are exemplified by barium glass, strontium oxide glass, lithium glass, silica barium sulfate, calcium phosphate, hydroxyapatite, fumed silicas such as Aerosil R972, Tullanox 300, and Cab-O-Sil PS-720, and various pigments known for use in dental compositions.

A preferred stabilizer is butylated hydroxytoluene (BHT).

When used as a base or liner, the composition will comprise by weight about 30-80%, preferably 40-70% resin monomers, 15-70%, preferably 20-40% inorganic fillers, about 0-10% fluoride salts, 0.05-2.0% photoinitiator, 0-2.0% reducing agent and optionally 0.1-1.0% stabilizers.

Preferred resin monomers used in the base composition are urethane dimethacrylate (UDMA) and PENTA.

Preferred inorganic fillers are $BaSO_4$ and barium glass.

A preferred bonding base composition comprises by weight about:
2-3% Dipentaerythritol pentaacrylate phosphate
53-63% Urethane dimethacrylate
0.1-0.5% Butylated hydroxytoluene
0.05-0.15% Lithium toluenesulfinate
0.15-0.35 Camphorquinone
0.9-1.3% N-morpholinoethylmethacrylate
0.2-0.6% Methyl diethanol amine
0.8-1.8% fumed Silica
18-22% Silanated barium sulfate
9.5-13.5% Silanated barium boron aluminum silicate glass
2.75-6.75% Sodium fluoride.

A specific base composition is illustrated in the following example.

## EXAMPLE I

| COMPOSITION OF LINER | |
|---|---|
| Raw Materials | % Wt. |
| Dipentaerythrito pentaacrylate phosphate(PENTA) | 2.51 |
| Urethane dimethacrylate[1] (UDMA) | 57.99 |
| Butylate hydroxy toluene (BHT) | 0.33 |
| Lithium toluenesulfinate (LTS) | 0.10 |
| Camphorquinone (CQ) | 0.24 |
| N-Morpholinoethylmethacrylate (MEM) | 1.11 |
| Methyl diethanol amine (MDEA) | 0.39 |
| Aerosil R972[2] | 0.70 |
| Tullanox 300[3] | 0.50 |
| Silanated barium sulfate[4] | 19.80 |
| Silanated Milled Corning Glass[5] | 11.54 |
| Sodium fluoride | 4.78 |

[1]Urethane dimethacrylate was prepared by reacting hydroxyl propyl methacrylate with 2,2,4-trimethylhexamethylene diisocyanate in a OH/NCO ratio of 1.05. Stannous octoate was used as catalyst.
[2]fumed silica (fluffy white powder).
[3]fumed silica (frothy white powder).
[4]silanated in-house, surface coated with 3-methacryloxypropyltrimethoxysilane (A174).
[5]Barium boron aluminum silicate glass mean particle size about 2.8 um.

## PROCEDURE

All additions and mixing was done under yellow light.

71.85 grams of PENTA, 1,656.9g UDMA, 9.3g BHT, 3.0g LTS, 6.75 CQ, 31.5g MEM, and 11.5g MDEA were weighed into a glass reactor. The mixture was mixed with high shear dispersator for 30 min at 55°C until a uniform solution was obtained.

20g Aerosil R972 and 14.28g Tullanex 300 were added to the resin mix by hand until the aerosil wetted out and the mixture was then mixed with a high shear dispersator at less than 55°C for 10 minutes.

The fillers, 565.5g 1% Silanated Barium Sulfate, 369.6g Silanated Milled Corning Glass and 136.5g sodium fluoride were added to the mixture by hand. When the fillers were wetted out, the mixture was mixed with a high shear dispersator at less than 55°C for 10 minutes or until a homogenous mix was formed.

The mixture was then passed through a colloid mill having a 0.01 inch setting.

The mixture was stored in a light tight container and degassed in a ROSS mixer at 45°C under 120 mm Hg for 45 minutes.

In the following table I, the physical properties of the base composition of the present invention are compared with VLC DYCAL®, a product of L. D. Caulk Corp., Milford, Delaware, a division of Dentsply International Inc., and G. C. Lining Cement, a product of G-C Industrial Dental Corp.

The table illustrates that the composition of the invention has higher compression strength than either commercial product used in the comparison, significantly higher tensile strength than the lining cements and significantly higher bonding strength to dentin than the DYCAL base composition.

The work time for the base material of the invention is at the command of the practitioner, and the cure is immediate on the application of actinic light.

There is no measurable pH of the resin material of the base composition as compared to the acidic properties of the G. C. lining cements and the basic properties of VLC DYCAL. The neutral pH of the base of the composition eliminates the practitioner's concern about what effect acidic or basic bases or liners have on the patients tooth or the filling materials used.

The tests described are standard tests which were carried out as described in ADA specification No. 8, 27 and 30.

The data relating to pH and solubility were obtained by following the instructions which follow.

TESTING METHOD FOR pH OF CALCIUM

HYDROXIDE BASE MATERIALS

I. Specimen Preparation - May be done 12-16 hours before testing will begin.

1. Set up the mold assembly. To do this place the squares of Mylar on the glass plates. Place the mold (outer brass ring and inner split teflon ring) on the Mylar. Have nylon string handy to the mold. Have clamps handy.

2. Weigh out the base and catalyst using the recommended weight ratio on a mix pad.

3. Mix the base and catalyst pastes together with a spatula until a homogenous mix is achieved (about 15 seconds). Then transfer the mixed material to the mold. Insert a piece of nylon string into the material so that the specimen may be suspended later. Then place another square of Mylar on top of the specimen followed by another glass plate. Press the plate down and then clamp the two plates together.

4. About 2 to 2 1/2 minutes after the start of mixing place the assembly in a 37° C oven.

5. Remove the assembly from the oven one hour after the start of mixing. Remove clamps, the glass plates, and the Mylar squares. Then carefully scrape the excess material from the outer brass ring and the inner teflon split ring. Once this is done run your fingernail around the edge be tween the brass ring and the teflon ring. This should allow you to carefully push out the inner teflon ring. Then the specimen can be separated from the teflon. Be very careful as the specimens are extremely brittle and break easily. They may be stored in an envelope until ready for use.

II. pH Testing Procedure

Fill a glass jar with 100 milliliters of distilled water. Test the initial pH of this water and record. Place a magnetic stir bar in the jar and place the jar on a stir plate. Immerse the specimen and start the stirrer. Insert the pH probe and take a reading. The remainder of the pH measurements should be taken at 1 hour, 3 hours, 7 or 8 hours, and 24 hours later.

The pH meter is standardized against buffers of pH 7 and pH 10 before use.

DETERMINATION OF SOLUBILITY

Sixty-second acid solubility for calcium hydroxide cements using 36% phosphoric acid.

This method is based on the procedure used by Hwas and Sandrik as published in the January, 1984 issue of the Journal of the American Dental Association (JADA).

Specimens are prepared in split teflon molds (20 mm diameter x 1.5 mm thick) which are fitted with outer brass rings for support. Mylar and glass plates are used to press the specimen. Nylon string is imbedded in the specimen.

Material is mixed according to the manufacturer's instructions, packed into the mold, pressed and clamped. At 2'00" from the start of mixing, the assembly is placed in a 37° C oven (100% humidity).

One hour from the start of mixing, the specimen is removed from the oven. The flash is carefully removed and the specimen is freed from the mold. Then the specimens are weighed on the four decimal place analytical balance. This is recorded as Reading A. The specimens are then immersed in 36% phosphoric acid for 60 seconds. Next, they are rinsed for 60 seconds with lukewarm tap water. Finally, they are blotted dry with Kimwipes and re-weighed. This weight is recorded as Reading B. The percent weight loss is calculated as:

$$\frac{(A-B)}{A} \times 100 = \% \text{ weight loss}$$

An average of three specimens should be reported.

The data indicates that the base of the invention has significantly better transverse strength and bond strength to human dentin than DYCAL, and has a comparable compressive strength.

The data in the table was obtained by procedures described by ADA specification No. 30 for compressive strength, International Standards Organization (ISO) #4049 for transverse strength and U.S. Patent 4,657,941 for the bond strength to human dentin.

TABLE I

| Physical Properties | GC Lining Cements* | VLC DYCAL | Invention Base Material |
|---|---|---|---|
| Work Time | 1′20″ | Unlimited | Unlimited |
| Set Time | 4′00″ | -- | -- |
| Thickness | 20 u | -- | -- |
| 24-hr. compressives (at break) | 10,855 psi (1,148) | 16,000 psi | 20,369 psi |
| 24-hr. Diametral Tensiles | 691 psi (28) | 5,500 psi | 4,500 psi |
| 24-hr. Solubility in $H_2O$ | 0.85% (0.005) | 0.2% | 0.0106% |
| 60 Second Acid Resistance | 2.65% (0.06) | 0 | 0.09% |
| Radiopacity (2 mm thickness) | 2-2.5 mm of Alumium | 3 mm of Aluminum | 3 to 4 mm of Aluminum |
| Immediate | 5.8 | 9.3 | -- |
| pH 1 hr. | 6.0 | 9.9 | -- |
| pH 3 hrs. | 6.7 | 10.1 | -- |
| pH 8 hrs. | 6.7 | 10.1 | -- |
| pH 24 hrs. | 6.9 | 10.4 | -- |
| Bond Strength to Dentin | @ 500 psi | < 100 psi | 400 to 1,000 psi |

*Standard Powder/Liquid ratio used of 1.2 grams powder to 1.0 gram of liquid as recommended by manufacturer.

. Standard deviation

The set time was done in a 37° C oven directly after mixing was completed. Film thickness measurements were also initiated directly after mixing due to the fast setting nature of the product. after mixing due to the fast setting nature of the product.

In the following table II, data relating to the compressive Strength, Transverse Strength and Bond Strength to human dentin of the base of the invention is compared with similar data obtained relating to the commercial composition DYCAL.

The control for each sample illustrates each kind of strength exhibited by a freshly prepared sample.

The "24 hour boiled" test illustrates the strength of each sample after being submerged in boiling water for 24 hours. "Thermocycled" test illustrates the strength of each sample after being placed alternately in a 10° C bath for one minute and a 50° C bath for one minute for a period of 24 hours.

TABLE II

| PHYSICAL PROPERTIES OF INVENTION BASE AND VLC DYCAL | | | | | | | | | |
|---|---|---|---|---|---|---|---|---|---|
| | Compressive Strength | | | Transverse Strength | | | Bond Strength and Human Dentin | | |
| Materials | Control | 24-Hr. Boiled | Thermocycled | Control | 24-Hr. Boiled | Thermocycled | Control | 24-Hr. Boiled | Thermocycled |
| Invention Base (112086) | 19,513 | 18,813 | 21,337 | 5,482 | 6,541 | 6,593 | 796 | 442 | 670 |
| VLC DYCAL | 21,396 | 18,117 | 20,900 | 3,196 | * | 2,233 | <100 | ** | ** |

*Specimen not suitable for testing
**Not determined because of the low initial bond strength exhibited by DYCAL.

EP 0 356 868 A2

The following table III, illustrates the fluoride release properties of the base composition of the invention. The fluoride release properties are an indication of how well the base composition will facilitate the prevention of dental caries when used.

The 112086 and 52886 samples described in the table were prepared as described in Example 1 except that no Tullanox 300 was used in the composition. It is believed that the differences in the fluoride eluting properties described were caused by slight differences in the method of preparing the compositions.

The fluoride release properties were measured as follows:

## METHOD OF FLUORIDE RELEASE MEASUREMENT

A chip of 20 mm diameter and 1 mm thick was prepared using the resin of the invention, curing the resin under photoflood lamp without a top cover for two minutes. As is normal with many actinic light cured polymers, oxygen inhibits cure on the surface of the polymer. After curing, the air inhibited layer was wiped off with Kimwipe. A tiny hole was drilled in the chip. The specimen was tied with a nylon thread, suspended in 10 ml deionized water in a plastic jar, capped with a lid, and stored in $37^\circ C$ oven for 1 week, or otherwise indicated. Water in each jar was decanted to a separate 30 ml plastic beaker. The specimen in the jar was washed with 1 ml deionized water, and the water rinse was added to the respective beaker. To the jar, 10 ml of fresh deionized water was added and put back in the $37^\circ C$ oven for the next measurement. The solution was diluted with 11 ml low level TISAB * solution and measured with fluoride electrodes. The fluoride concentrations are reported in the attached tables.

*TISAB - Total Ionoic Strength Adjustor Buffer

8

## TABLE III

### FLUORIDE RELEASE MEASUREMENTS

| Week No. | Concentration (ppm) 52886 | Concentration (ppm) 112086 | Cumulative Concentration (ppm) 52886 | Cumulative Concentration (ppm) 112086 |
|---|---|---|---|---|
| 1 | 27.131 | 36.300 | 27.131 | 36.300 |
| 2 | 5.437 | 6.370 | 32.568 | 42.670 |
| 3 | 2.490 | 6.156 | 35.057 | 48.826 |
| 4 | 15.432 | 6.156 | 50.489 | 54.982 |
| 5 | 19.544 | 6.156 | 70.033 | 61.137 |
| 6 | 14.747 | 6.156 | 84.780 | 67.293 |
| 7 | 15.182 | 6.528 | 99.962 | 73.822 |
| 8 | 13.322 | 6.928 | 113.284 | 80.749 |
| 9 | 13.322 | 6.460 | 126.605 | 87.209 |
| 10 | 12.028 | 5.654 | 138.634 | 92.863 |
| 11 | 10.984 | 5.611 | 149.618 | 98.474 |
| 12 | 10.360 | 4.876 | 159.977 | 103.350 |
| 13 | 10.159 | 4.929 | 170.136 | 108.279 |
| 14 | 10.051 | 5.051 | 180.187 | 113.330 |
| 15 | 8.661 | 4.067 | 188.848 | 117.397 |
| 16 | 8.313 | 3.961 | 197.161 | 121.358 |
| 17 | 7.904 | 3.859 | 205.065 | 125.217 |
| 18 | 6.814 | 3.653 | 211.878 | 128.870 |
| 19 | 5.569 | 3.282 | 217.447 | 132.151 |
| 20 | 6.238 | 3.020 | 223.685 | 135.171 |
| 21 | 5.317 | 3.066 | 229.003 | 138.238 |
| 22 | 6.241 | 3.125 | 235.244 | 141.362 |

## FLUORIDE RELEASE MEASUREMENTS
### BASE/LINER

| Week No. | Concentration (ppm) 52886 | 112086 | Cumulative Concentration (ppm) 52886 | 112086 |
|---|---|---|---|---|
| 23 | 5.373 | 2.940 | 240.617 | 144.302 |
| 24 | 5.868 | 2.435 | 246.485 | 146.737 |
| 25 | 4.245 | 2.254 | 250.730 | 148.990 |
| 26 | 4.785 | 2.396 | 255.515 | 151.386 |
| 27 | 4.633 | 2.252 | 260.148 | 153.638 |
| 28 | 4.500 | 1.957 | 264.648 | 155.595 |
| 29 | 4.500 | 1.841 | 269.148 | 157.436 |
| 30 | 4.500 | 1.978 | 273.648 | 159.414 |
| 31 | 4.500 | 1.929 | 278.148 | 161.344 |
| 32 | 4.213 | 1.923 | 282.361 | 163.267 |
| 33 | 4.300 | 1.923 | 286.661 | 165.190 |
| . . . | . . . | . . . | . . . | . . . |
| 50 | 2.470 | 1.923 | 337.330 | 197.881 |
| 51 | 2.288 | 1.923 | 339.618 | 199.804 |
| 52 | 2.423 | 1.923 | 342.041 | 201.727 |
| 53 | 2.073 | | 344.114 | |
| 54 | 2.191 | | 346.305 | |
| 55 | 1.999 | | 348.303 | |

It is believed that the properties described in the tables illustrate that the base composition of the invention is Biologically kind and non-toxic to the pulp which allows for pulpal recovery when the base is used; provides a milder histological response as compared with glass ionomer and zinc phosphate base materials which increases patient comfort; bonds to dentin and overlying resin materials without need for etch which provides an integrally bonded, secure restoration; and because the composition is a one-component, visible light-cured composition, the practitioner controls the work time and does not have to mix the base prior to use; and the cured resin has excellent physical properties which make it possible for the base to withstand condensing and masticatory forces under restorative materials, and to resist attack from fluids in the oral environment.

Optionally, the base may be made radiopaque which assists in radiographic diagnosis.

In the embodiment which contains significant amount of fluoride it has been found that the fluoride is released over protracted period without adverse effect on physical properties of the base.

While present embodiments of the invention and method of practicing the same have been illustrated and described, it will be recognized by those skilled in the art that this invention may be otherwise variously embodied and practiced within the scope of the following claims.

## Claims

1. A method of lining or basing a prepared tooth cavity comprising coating said tooth cavity with dental resin composition which comprises at least one adhesive promoting agent.

2. The method of Claim 1 comprising coating said prepared tooth cavity with a base composition comprising elutable fluoride.

3. The method of Claim 1 comprising coating said tooth cavity with a resin composition comprising elutable sodium fluoride.

4. The method of Claim 2 comprising coating said prepared tooth cavity with a base comprising by weight about
    (a) 40-70% resin monomers
    (b) 20-70% inorganic fillers
    (c) 2.5-7.0% NaF
    (d) 0.05-1.5% photoinitiator, and
    (e) 0.2-2.0% amine reducing agent

5. The method of Claim 5 comprising coating said prepared tooth cavity with a bonding base comprising by weight about
2-3% Dipentaerythritol pentaacrylate phosphate
53-63% Urethane dimethacrylate
0.1-0.5% Butylated hydroxytoluene
0.05-0.15% Lithium toluenesulfinate
0.15-0.35 Camphorquinone
0.9-1.3% N-morpholinoethylmethacrylate
0.2-0.6% Methyl diethanol amine
0.8-1.8% fumed Silica
18-22% Silanated barium sulfate
9.5-13.5% Silanated barium boron aluminum silicate glass
2.75-6.75% Sodium fluoride.

6. A composition for treating a prepared tooth cavity comprising a dental resin base composition which comprises at least one adhesive promoting agent.

7. The composition of Claim 7 comprising a base composition comprising elutable fluoride.

8. The composition of Claim 8 comprising a resin composition comprising elutable sodium fluoride.

9. The composition of Claim 8 comprising a bonding base comprising by weight about
    (a) 40-70% resin monomers
    (b) 20-70% inorganic fillers
    (c) 2.5-7.0% NaF
    (d) 0.05-1.5% photoinitiator, and
    (e) 0.2-2.0% amine reducing agent

10. The composition of Claim 11 comprising a bonding base comprising by weight about
2-3% Dipentaerythritol pentaacrylate phosphate
53-63% Urethane dimethacrylate
0.1-0.5% Butylated hydroxytoluene
0.05-0.15% Lithium toluenesulfinate
0.15-0.35 Camphorquinone
0.9-1.3% N-morpholinoethylmethacrylate
0.2-0.6% Methyl diethanol amine
0.8-1.8% fumed Silica
18-22% Silanated barium sulfate
9.5-13.5% Silanated barium boron aluminum silicate glass
2.75-6.75% Sodium fluoride.